# EUROPEAN PATENT APPLICATION

(11) **EP 1 994 938 A1**
(43) Date of publication of application: **26.11.2008**
(21) Application number: 07010061.5
(22) Date of filing: 21.05.2007
(51) Int. Cl.: A61K 38/16, A61K 47/40, A61P 31/04, A61P 31/12, A61P 33/00

(54) **New lipoglycodepsipeptide compositions**

(71) Applicant: Combinature Biopharm AG, 13125 Berlin (DE)
(72) Inventor: Pelzer, Stefan, Dr., 13189 Berlin (DE); Vente, Andreas, Dr., 10553 Berlin (DE)
(74) Representative: Webster, Jeremy Mark

(57) **Abstract**

The invention relates to a pharmaceutical composition comprising as an active agent a lipoglycodepsipeptide in a physiologically effective dose and a cyclodextrin or a cyclodextrin derivative.

## Description

### Field of the invention.

The invention relates to new pharmaceutical compositions containing lipoglycodepsipeptides, to the use of such compositions, to methods for the production thereof and to the use thereof as drugs.

### Background of the invention and prior art.

Secondary metabolites, which are produced by living organisms, in particular microorganisms, and the chemical variants derived therefrom are successfully used as active agents in medicine. Particularly for the control of infectious diseases, the use of secondary metabolites has proven effective. A large portion of the antibiotics used today were isolated from bacteria in the soil, the so-called actinomycetes. Due to the development of resistances against the respectively used drugs, there is a permanent demand of new antibiotic active agents with novel activity mechanisms. In spite of their excellent antibiotic and other pharmacological properties, for many secondary metabolites the use as a drug is at last unsuccessful because of poor tolerance caused by toxic properties for man, poor stability or poor systemic absorption.

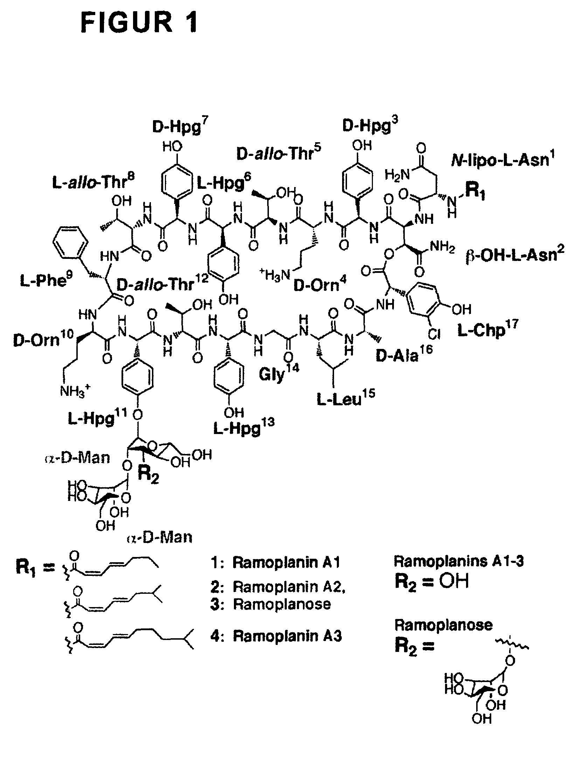

The antibiotic ramoplanin is a natural compound representing the structural class of lipoglycodepsipeptides. Ramoplanin is usually present as a complex mixture of closely related molecules, the primary variants being A1, A2 and A3 after fermentation of an Actinoplanes spp producer strain. Isolation of ramoplanin is described in Cavalleri et al., 1984, J. Antibiot,. 37, 309-317. The main products A1, A2 and A3 differ in the length of the N-terminal acyl chain attached to Asn1. Ramplanins A1-3 consists of 49 membered macrocyclic depsipeptide containing 17 constituents with 7 D-amino acids joined by a lactone linkage between amino acid 2 and 17 (see Fig 1). A1-A3 contain 2 D-mannosyl carbohydrates.

Ramoplanin inhibits the peptidoglycan biosynthesis of gram-positive bacteria. The bactericidal mechanism of action is unique and a result of the direct inhibition of bacterial transglycosylase by binding as a dimer to lipid II (He et al., 2003 JACS, 125, 8736-8737). Thus ramoplanin is active against a wide range of aerobic and anaerobic gram-positive bacteria with no cross resistance between ramoplanin and vancomycin or teicoplanin. Excellent in vitro activity of ramoplanin [general MICs (Minimal Inhibition Concentrations) are below 1 µg/ml] has been documented against Streptococcus spp., Staphylococcus spp. and Enterococcus spp. including antibiotic resistant strains, like vancomycin-resistant Enterococci (VRE) and methicillin resistant S. aureus (MRSA). Moreover, ramoplanin has demonstrated in vitro activity against a wide range of anaerobic bacteria, like C. difficile. (Karver et al., 2005, Annals of Pharmacotherapy, 39, 863-868).

Since orally administered ramoplanin is not systemically absorbed but has high concentration in feces, clinical applications of oral formulations are limited to the treatment of gastrointestinal carriage, nasal staphylococcal carriage and antibiotic-associated diarrhea (McCafferty, Peptide Science: 66, 261-284). The current clinical development status of ramoplanin can be summarized as follows: i. Phase III clinical trials for the treatment of C. difficile-associated diarrhea (CDAD); ii. Phase III clinical trials for the prevention of systemic nosocomial infections caused by VRE in the intestinal tract. Moreover a topical formulation has been developed and is currently tested in Phase II clinical trial for the eradication of nasal carriage of MRSA.

In conclusion, ramoplanin can be seen as a valuable potent antibiotic of a new structural class without any cross-resistance to other antibacterial agents.

Beside the fact that ramoplanin is not orally absorbed the main disadvantage for the broad application is the fact that this antibiotic cannot be administered parenterally. Two main reasons are responsible for the failure. First, ramoplanin is instable in the blood stream caused by a rapid hydrolysis of the depsipeptide ester. Synthesis of analogues with amide linkages helped to overcome this problem. Accordingly, total synthesis of a ramoplanin analogue in which the backbone ester was replaced with an amide retained or enhanced antimicrobial potency and physical stability (Rew et al., 2004, JACS, 126: 1041-1043; Chen et al., 2004 JACS, 126: 7462-7463). Second, parenteral administration of ramoplanin caused swelling and progressive necrotiazation at the injection site, and hemolysis as revealed by urine discoloration. To overcome this problem an injectable formulation for ramoplanin based on fat emulsions was suggested (US 6,720,305 and WO00/06119).

Fat formulations used are complex emulsions comprising an oil phase, emulsifiers and additives dispersed in water for injection. In the patent mentioned above it is stated that in rat tolerability tests ramoplanin in a concentration of 10 mg/ml (20mg/kg) could be administered in emulsions composition containing Intralipid, if the oil phase is at least 4% (w/vol) of the total formulation. If the concentration was reduced to 1 mg/ml (10 mg/kg) the oil phase has to be at least 0.2% (w/vol) to prevent side effects. In septicaemia mouse models the delivered drug was effective in a formulation with an oil phase of 8%.

The formulation procedure prior to administration described according to US 6,720,305 is complicated and time consuming, since one has to obtain a homogenous dissolution after the reconstitution of the fat emulsion to glucose and/or the dissolved ramoplanin. Furthermore, the emulsion tends to separate, which leads to low shelf lifes.

In other contexts it is known in the art to use cyclodextrins in pharmaceutical compositions. Due to their circular structure, cyclodextrins have a hydrophilic exterior and hydrophobic inner pocket. By enclosing in particular hydrophobic sections of the molecules, cyclodextrins can achieve a "molecular encapsulation" or "masking" of active agents, which are used for instance as a protective envelope of sensitive molecules in cosmetic and pharmaceutical formulations. Thereby, improved solubilities of substances, but also reduced toxicities, such as for instance a reduction of the hemolytic properties of molecules (J. Pharmacobiodyn. 1983 6(6): 408-14. Protective mechanism of beta cyclodextrin for the hemolysis induced with phenothiazine neuroleptics in vitro. Irie T, Sunada M, Otagiri M, Uekama K.) are obtained.

### Technical object of the invention.

It is therefore the technical object of the invention to provide a new pharmaceutical composition comprising a ramoplanin compound, wherein the tolerance is improved, wherein the physiological effectiveness is maintained, even at very high concentrations like during infusions for short times at the place of application, wherein only little toxic side effects are encountered, and wherein the shelf-life is improved.

### Basics of the invention and embodiments.

For achieving this technical object, the invention teaches a pharmaceutical composition comprising as an active agent an antibacterially active ramoplanin or ramoplanin derivative in a physiologically effective dose and a physiologically tolerated cyclodextrin or a cyclodextrin derivative.

The invention is based on the surprising finding that specially by using cyclodextrins or cyclodextrin derivatives not only a reduction of the hemolytic properties and the injection side swelling of antibiotically active ramoplanin is achieved, but that rather at the same time the antibiotic effect of the ramoplanin is maintained, if not enhanced.

Preferably, the lipoglycodepsipeptide has a structure according to formula I 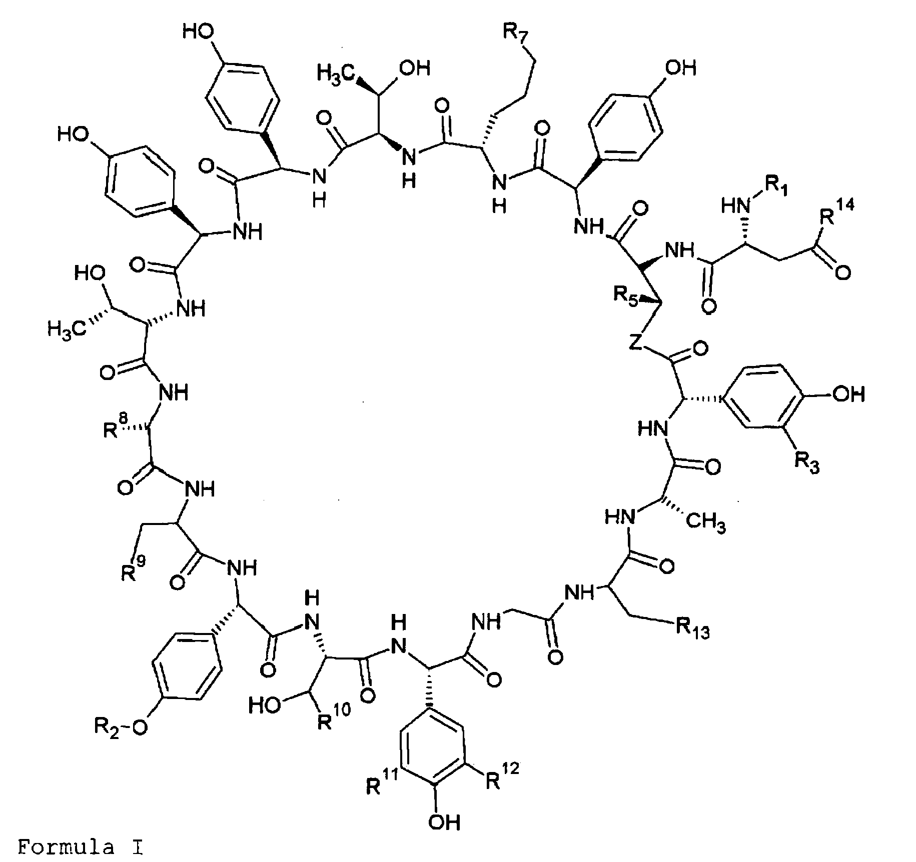
wherein R1 is -H, or a saturated or unsaturated, branched or linear aliphatic, or aromatic, or aliphatic/aromatic acyl residue with 2 to 30 carbon atoms,
wherein R2 is -H or any glycone,
wherein R3 is -H or halogen,
wherein R5 is -H, -CH₃, -COOH, -COO⁻, or -(CO)-NH₂,
wherein R8 is -CH₂-C₆H₅ or -CH₂-CH₂-CH₂-NH-(CO)-NH₂
wherein R9 is -CH₂-CH₂-R6 or 2-iminoimidazolidin-4-yl,
wherein R10 is -H or -CH₃,
wherein R11 and R12 are independently of each other - H or halogen,
wherein R13 is -CH(CH₃)₂ or 2-iminoimidazolidin-4-yl,
wherein R14 is -NH₂ or -OH,
wherein R6 and R7 are independently of each other - NH₃⁺, -NH₂, -NHR4, - -NH₂⁺-R4, wherein R4 is -Ac, - C(NH)NH₃⁺, or saturated or unsaturated, branched or linear C1-C12-alkyl,
and
wherein Z is -O-, -NH-, or -NH-CH₂- (amide bond).

A ramoplanin or ramoplanin derivative is obtained, if R3 is -Cl, R5 is -(CO)NH₂, R8 is -CH₂-C₆H₅, R9 is -CH₂-CH₂-R6, R10 is -CH₃, R11 and R12 are -H, R13 is -CH(CH₃)₂, and R14 is -NH₂. An Enduracidin or Enduracidin derivative is obtained, if R3 is -H, R5 is -CH₃. R8 is -CH₂-CH₂-CH₂-NH-(CO)-NH₂, R9 is 2-iminoimidazolidin-4-yl, R10 is -H, R11 and R12 are -Cl, R13 is 2-iminoimidazolidin-4-yl, and R14 is -OH. Further comprised in the invention are Janiemycins, which have a very similar backbone structure. In the following general specification and the claims the term "ramoplanin" stands for all compounds falling under the general formula I.

The glycone is preferably a mono-, di-, or linear or branched trisaccharide. It may in particular be selected from the group consisting of "alpha-D-mannopyranosyl, 2-O-alpha-D-mannopyranosyl-alpha-D-mannopyranosyl, and 2,3-O-di(alpha-D-mannopyranosyl)-D-mannopyranosyl".
R1 may have any of the structures
   -(CO)-CHR11-CHR12-CHR13-CHR14-R5,
   -(CO)-CR11=CR12-CHR13-CHRI4-R5,
   -(CO)-CHR11-CR12=CR13-CHR14-R5,
   -(CO)-CHR11-CHR12-CR13=CR14-Rl5,
   -(CO)-CR11=CR12-CR13=CR14-R15,
   wherein R11, R12, R13, and R14 each independently are -H, or C1-C5-alkyl, and wherein R15 is branched or linear C1-C10-alkyl,
   or the structure
   -(CO)-A-R16,
   wherein A is a bond or linear or branched C1-C4-alkylene or C2-C4-alkylidene, optionally containing a double bond, and wherein R16 is linear or branched C1-C4-alkoxy, optionally comprising a double bond and/or being substituted by 1 to 3 halogen atoms, or
   wherein R16 is phenyl, optionally substituted with 1 to 3 substituents independently selected from halogen, cyano, nitro, C1-C4-alkyl, optionally substituted with 1 to 3 halogen atoms, C1-C4-alkoxy, optionally substituted with 1 to 3 halogen atoms, C1-C4-thioalkyl, optionally substituted with 1 to 3 halogen atoms, phenyl, C1-C10-phenylalkyl, phenoxy, C1-C10-phenoxylalkyl, wherein the phenyl-portions may be substituted with halogen, cyano, nitro, C1-C4-alkyl, optionally substituted with 1 to 3 halogen atoms, C1-C4-alkoxy, optionally substituted with 1 to 3 halogen atoms, C1-C4-thioalkyl, optionally substituted with 1 to 3 halogen atoms, or
   wherein R16 is naphthyl, optionally substituted with 1 to 3 substituents independently selected from C1-C4-alkyl, optionally substituted with 1 to 3 halogen atoms, C1-C4-alkoxy, optionally substituted with 1 to 3 halogen atoms, or
   wherein R16 is phenoxy, optionally substituted with 1 to 3 substituents independently selected from halogen, cyano, nitro, C1-C4-alkyl, optionally substituted with 1 to 3 halogen atoms, C1-C4-alkoxy, optionally substituted with 1 to 3 halogen atoms, Cl-C4-thioalkyl, optionally substituted with 1 to 3 halogen atoms, phenyl, C1-C10-phenylalkyl, phenoxy, C1-C10-phenoxylalkyl, wherein the phenyl-portions may be substituted with halogen, cyano, nitro, C1-C4-alkyl, optionally substituted with 1 to 3 halogen atoms, C1-C4-alkoxy, optionally substituted with 1 to 3 halogen atoms, C1-C4-thioalkyl, optionally substituted with 1 to 3 halogen atoms, C1-C4-thioalkyl, optionally substituted with 1 to 3 halogen atoms or
   wherein R16 is naphthoxy, optionally substituted with 1 to 3 substituents independently selected from C1-C4-alkyl, optionally substituted with 1 to 3 halogen atoms, C1-C4-alkoxy, optionally substituted with 1 to 3 halogen atoms,
   wherein when A is a bond, R16 is neither alkoxy, phenoxy, nor naphthoxy.

C1-C4-alkyl comprises methyl, ethyl, propyl, n-butyl, 1-methylpropyl, 2-methylpropyl, tert.-butyl, etc. Halogen atom comprises -F, -Cl, -Br, -I.

The term linear or branched C1-C4-alkylene and C2-C4-alkylidene, optionally comprising one double bond comprises all possible permutations within the limitations of the number of carbon atoms.

R1 is preferably selected from the group of the following acyl residues:
-CO-CH₃
-CO-CH=CH-CH=CH-CH₂-CH(CH₃)₂,
-CO-CH=CH-CH=CH-CH₂-CH₂-CH₃,
-CO-CH=CH-CH=CH-CH₂-CH₂-CH(CH₃)₂,
-CO-CH=CH-CH=CH-CH₂-CH₂-CH₂-CH₂-CH(CH₃)₂,
-CO-CH=CH-CH=CH-CH₂-CH₂-CH₂-CH₂-CH(CH₃)-CH₂-CH₃,
-CO-CH₂-CH₂-CH₂-CH₂-CH₂-CH₂-CH₃,
-CO-CH₂-CH₂-CH₂-CH₂-CH₂-CH(CH₃)₂, and
-CO-CH₂-CH₂-CH₂-CH₂-CH₂-CH₂-CH(CH₃)₂.

Optionally, the pharmaceutical composition includes two or more different ramoplanin compounds, preferably each, but at least one, in a physiologically effective dose. Then it is a combination composition or a wide band composition.

In detail, the ramoplanin compound may be present in a free form or as an acid addition salt thereof.

The ramoplanin compound is comprised in the pharmaceutical composition preferably in a total amount (referred to the amount of all employed ramoplanin) from 0.01 to 80 wt.%, in particular from 0.05 to 50 wt.%, preferably from 0.1 to 30 wt.%, wherein the amount figures are referred to the completed composition.

In principle, all physiologically tolerated cyclodextrins and cyclodextrin derivatives can be employed. Cyclodextrins are cyclic oligosaccharides, which are composed of alpha-1,4-linked glucose components. Usually, six to eight glucose components (α, β, or γ-cyclodextrin) are connected with each other in a cyclodextrin molecule. Besides the naturally occurring, unmodified cyclodextrins, there is a large number of chemically modified cyclodextrin derivatives, which are physiologically tolerated and can be used for the purpose of the invention. The cyclodextrin or cyclodextrin derivative preferably is an α or β-cyclodextrin and may in particular have the general formula II, 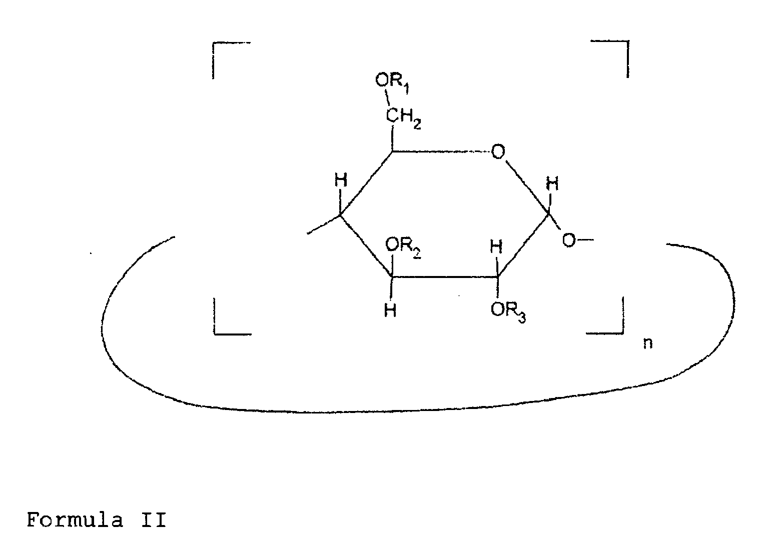 wherein R1, R2, and R3 may be identical or different and any physiologically tolerated residue, preferably -H, C1-C8 alkyl, -SO₂OH, -PO(OH)₂, or -CO-R4 with R4 = C1-C8 alkyl, wherein the C1-C8 alkyl may be substituted once or more than once at identical or at different C atoms with - OH, -COOH, -CONHR5, -NHCOR6, -SO₂OH, -PO(OH)₂, or tetrazol-5-yl with R5 = -H or C1-C4 alkyl and R6 = carboxylphenyl, wherein n = 6, 7 or 8, wherein R1, R2 and R3 may be randomized in different glucopyranose units, wherein an oxygen atom or several oxygen atoms of the glucopyranose units, in particular the oxygen atom at C6, may be substituted by sulfur atoms, including physiologically tolerated salts of such cyclodextrins. Preferably, the glucopyranose units are α-D or α-L-glucopyranose units. C1-C8 alkyl comprises in particular methyl, ethyl, propyl, isopropyl, butyl, isobutyl and tertiary butyl. On average, 1 to 3, preferably 1 to 2 of the residues R1, R2 and R3 may be different from H. Preferably, in particular R1 is different from -H. 1, 2, 3, 4, 5, 6, or if applicable 7 of the residues R1 of a cyclodextrin molecule may be different from -H. R2 and R3 may then be -H. In addition, however, 1, 2, 3, 4, 5, 6, or if applicable 7 of the residues R3 of a cyclodextrin molecule may also be different from -H.

In detail, the cyclodextrin or cyclodextrin derivative may be selected from the group consisting of "α-cyclodextrin, β-cyclodextrin, hydroxy-(C1-C8 alkyl)-α-cyclodextrin, hydroxy-(C1-C8 alkyl)-β-cyclodextrin, (2-hydroxypropyl)-β-cyclodextrin, (2-hydroxypropyl)-α-cyclodextrin, sulfo-(C1-C8 alkyl)-ether-α-cyclodextrin, sulfo-(C1-C8 alkyl)-ether-β-cyclodextrin, sulfobutylether-α-cyclodextrin, sulfobutylether-β-cyclodextrin". For the derivatives, in particular the residue at the oxygen atom of the C6 atom is different from -H.

The cyclodextrin or cyclodextrin derivative may be present in the pharmaceutical composition in an amount from 0.01 to 99 wt.%, in particular from 0.1 to 95 wt.%, preferably 5 to 95 wt.%, referred to the undissolved solid drug.

Preferably, the ramoplanin in the pharmaceutical composition is mixed with the cyclodextrin or cyclodextrin derivative in a molar ratio ramoplanin/cyclodextrin from 10:1 to 1:500, preferably 5:1 to 1:50, most preferably 1:1 to 1:30, optionally under addition of additional and/or auxiliary substances in galenically common additions.

Usually, the pharmaceutical composition will comprise further additional and/or auxiliary substances, in particular galenic auxiliary substances, the selection of which depends from the selected form of administration. The galenic preparation of the pharmaceutical composition according to the invention may be made in a way being usual for this technology. As counter ions for ionic compounds may for instance be used Ca++, CaCl+, Na+, K+, Li+ or cyclohexylammonium or Cl-, Br-, acetate, trifluoroacetate, propionate, lactate, oxalate, malonate, malate, maleinate, citrate, benzoate, salicylate etc. Suitable solid or liquid galenic forms of preparation are instance granulates, powders, dragees, tablets, (micro) capsules, suppositories, syrups, juices, suspensions, emulsions, drops or injectable solutions (IV, IP, IM, SC) or fine dispersions (aerosols), forms of preparation for dry powder inhalation, transdermal systems, and preparations with protracted release of active ingredient, for the production of which usual means are used, such as carrier substances, explosives, binding, coating, swelling, sliding or lubricating agents, tasting agents, sweeteners and solution mediators. As auxiliary substances are named here magnesium carbonate, titanium dioxide, lactose, mannite and other sugars, talcum, milk protein, gelatin, starch, cellulose and derivatives, animal and vegetable oils such as cod-liver oil, sunflower, peanut or sesame oil, polyethylene glycols and solvents, such as sterile water and mono or multivalent alcohols, for instance glycerin. A pharmaceutical composition according to the invention can be produced by that at least one combination of substances used according to the invention is mixed in a defined dose with a pharmaceutically suitable and physiologically well tolerated carrier and possibly further suitable active, additional or auxiliary substances, and is prepared in the desired form of administration. Preferred are solutions for injection in the usual preparation.

As dilution agents, polyglycols, ethanol, water and buffer solutions can be used. Suitable buffer solutions are for instance N,N'-dibenzylethylendiamine, diethanolamine, ethylendiamine, N-methylglucamine, N-benzylphenethylamine, diethylamine, phosphate, sodium bicarbonate, or sodium carbonate. It is however also possible not to use any dilution agent at all.

Physiologically tolerated salts, whether of the ramoplanin, or of the cyclodextrins or cyclodextrin derivatives, are salts with inorganic or organic acids, such as hydrochloric acid, sulfuric acid, acetic acid, citric acid, p-toluolsulfonic acid, or with acids of the above-mentioned ionic groups, or with inorganic or organic bases, such as NaOH, KOH, Mg(OH)₂, diethanolamine, ethylendiamine, or with amino acids, such as arginine, lysine, glutamine acid etc., or with inorganic salts, such as CaCl₂, NaCl or the free ions thereof, such as Ca²⁺, Na⁺, Cl⁻, SO₄²⁻ or combinations thereof. They are also produced by using standard methods.

In detail, a pharmaceutical composition according to the invention may comprise: A) 0.01 to 80 wt.%, in particular 0.05 to 50 wt.%, preferably 0.1 to 30 wt.% ramoplanin, B) 0.01 to 99 wt.%, in particular 0.1 to 95 wt.%, preferably 5 to 95 wt.% cyclodextrin or cyclodextrin derivative, C) 0.1 to 99.8 wt.%, in particular 1 to 80 wt.%, preferably 1 to 50 wt.% additional and/or auxiliary substances and optionally dilution agents, wherein the components A) to C) add up to 100 % and wherein the ramoplanin in a physiologically effective dose is mixed with the cyclodextrin or cyclodextrin derivative in a molar ratio ramoplanin/cyclodextrin from 10:1 to 1:500, preferably 5:1 to 1:50, most preferably 1:1 to 1:30, optionally under addition of additional and/or auxiliary substances in galenically usual additions.

As far as above and below statements are made with regard to wt.%, molar ratios and/or doses, they always refer to the so-called free acid of the ramoplanin, provided it is used in a salt form. Counter ions of salt forms are not taken into account, but are substituted by the atomic weight of hydrogen. Counter ions are rather used as additional or auxiliary substances.

The invention relates further to the use of a pharmaceutical composition according to the invention for the production of a drug for the treatment and/or prophylaxis of viral, bacterial and/or parasitary infectious diseases and/or of fungal diseases. Examples of such diseases or applications are: infections of the respiratory tract, infections of the skin and the soft parts, infections of the urinary tract, infections of the gallbladder tract, sepsis, endocarditis, meningitis, op prophylaxis, wound infections or intraabdominal infections.

It is possible that the drug is galenically prepared for the oral administration, but preferred is the preparation for parenteral application, e.g. for injection.

The invention furthermore relates to a method for the treatment of a bacterial, viral or parasitary infectious disease or a fungal disease, wherein a person, which has fallen ill with the disease or is in danger of falling ill therewith, is administered a physiologically effective dose of a drug according to the invention. The daily dose may be from 1 to 50,000 mg, preferably 50 to 30,000 mg, most preferably from 100 to 20,000 mg ramoplanin over a period from 1 to 60 days, preferably 1 to 30 days.

Packing units with a multitude of administration units may be provided, wherein every administration unit is prepared for an administration within the above treatment plan. For example, a packing unit may contain n1 = 5 to n2 = 500 administration units, wherein every administration unit contains ml = 1/5 to m2 = 1 daily dose of the ramoplanin. The packing unit is then prepared for a treatment plan, which provides 1 to 5 administrations per day over a period of o1 to o2 days, wherein o is then calculated by o1 = n1 * m2 and o2 = n2 * ml, or o and m are given and n is calculated as n = o/m.

In the following, the invention is explained in more detail by comparative examples and not limiting examples according to the invention.

### Example 1: Minimization of the hemolysis induced by ramoplanin by the addition of cyclodextrins.

Ramoplanin was dissolved in a concentration of 10,000 mg/l in 0.9 % NaCl solution. 20 µl of this stock solution was mixed with 20 µl stock solution of (2-Hydroxypropyl)-γ-cyclodextrin (HP-γ-CD), (2-Hydroxypropyl)-β-cyclodextrin (HP-β-CD), sulfobutylether-β-cyclodextrin (SBE-β-CD) or α-cyclodextrin (α-CD) dissolved in 0.9 % NaCl at a concentration of 4 wt.%. After pre-incubation for 1 hour at ambient temperature, 40 µl of fresh venal human blood was added, mixed carefully and incubated at 37 °C for 180 min. As a negative control, 40 µl of fresh venal blood were prepared with 40 µl 0.9 % NaCl, as a standard for the complete hydrolysis a mixture von 40 µl of fresh venal human blood were prepared with 40 µl water. Subsequently, the degree of the hemolysis induced by the incubation was determined as follows: The samples were cautiously mixed either with 1 ml water (standard for the complete hydrolysis) or with 1 ml 0.9 % NaCl. After centrifugation of the samples at 2,500 RFC (5 min), the absorption of the supernatant was determined in a spectral photometer at 540 nm. Before the measurement of the samples, the spectral photometer was calibrated with the respective negative control described above. For the determination of the degree of hemolysis of the different reaction batches, the measured value of the standard for the complete hydrolysis was set to 100 %. The measured values of the different reaction batches were related to the value of this standard and calculated in percent. Table 1 shows the result of the hemolysis test with ramoplanin and different cyclodextrins performed with human blood. The listed concentrations of the cyclodextrins (in % wt./vol.) and of the ramoplanin (in mg/l, active compound) refer to the final concentrations in the reaction batch.

**Table 1**

| Hemolytic activity as a function of ramoplanin concentration (2,500 mg/1) in presence of different cyclodextrins (1 wt.%) in vitro (in %) | |
|---|---|
| Cyclodextrin conc. | Ramoplanin concentration in mg/l 2500 |
| without cyclodextrin addition | 45% |
| 1 % (wt./vol.) α-CD | 37% |
| 1 % (wt./vol.) HP-β-CD | 20% |
| 1 % (wt./vol.) HP-γ-CD | 43% |
| 1 % (wt./vol.) SBE-β-CD | 3% |

The determination of the content of free hemoglobin in the serum showed that after pre-incubation with 1% HP-γ-CD and 1% α-CD no significantly reduction the hemolytic potential of ramoplanin at a concentration of 2,500 mg/l could be determined whereas the addition of 1% HP-β-CD resulted in a reduction of the hemolysis induced by ramoplanin. The addition of 1% SBE-β-CD minimized the hemolysis induced by ramoplanin to a low level.

### Example 2: Minimization of the hemolysis induced by lipoglycodepsipeptides by the addition of different concentrations of sulfobutylether-β-cyclodextrin (SBE-β-CD).

This example shows the effect of different molar ratios of cyclodextrins to lipoglycodepsipeptides on the hemolytic effect induced by the lipoglycodepsipeptides. Herein, ramoplanin serves as an example molecule for the antibiotics of the class of the lipoglycodepsipeptides and sulfobutylether-β-cyclodextrin (SBE-β-CD) as an example molecule for the cyclodextrins.

This example shows the effect of different molar ratios of cyclodextrins to lipoglycodepsipeptides on the hemolytic effect induced by the lipoglycodepsipeptides. Herein, ramoplanin serves as an example molecule for the antibiotics of the class of the lipoglycodepsipeptides and sulfobutylether-β-cyclodextrin (SBE-β-CD) as an example molecule for the cyclodextrins.

Ramoplanin was dissolved in a concentration of 10,000 mg/l and 2,000 mg/l in 0.9 % NaCl solution. To different batches of this stock solution were added different SBE-β-CD concentrations, so that the following molar ratios (SBE-β-CD : ramoplanin) were generated: 0:1; 1:1; 2.5:1; 5:1; 10:1. The pre-incubation and the execution of the test for the determination of the hemolytic activity with fresh venal human blood were made according to Example 1 with an incubation period of 15 instead of 180 minutes. The results of this test are shown in table 2. The content of ramoplanin (in mg/l) refers to the final concentration of the free acid of ramoplanin in the reaction batch. The molar ratios refer to the free acids of ramoplanin and SBE-β-CD.

**Table 2**

| Reduction of the hemolysis induced by 2,500 mg/l ramoplanin by SBE-β-CD in vitro (in %) | |
|---|---|
| Molar ratio SBE-β-CD : ramoplanin | Reduction of the induced hemolysis |
| 0 : 1 | 51% |
| 1 : 1 | 60% |
| 2.5 : 1 | 44% |
| 5 : 1 | 18% |
| 10 : 1 | 6% |

### Example 3: Effects of the additions of cyclodextrins on the antibiotic activity of Ca₂Cl₂ ramoplanin B.

The effects of cyclodextrins on the antibiotic activity of ramoplanin were investigated by in vitro experiments about the growth inhibition of Gram-positive bacteria. Herein, the minimum inhibitory concentration for the growth inhibition was determined by cultivation of the bacteria in Ca²⁺ adjusted Mueller Hinton broth (broth microdilution method) according to the CLSI (previously NCCLS) rules (National Committee for Clinical Laboratory Standards. 2003. Methods for dilution antimicrobial susceptibility tests for bacteria that grow aerobically; approved standard - 6th ed. Document M7-A6. Clinical and Laboratory Standards Institute, Wayne, PA, USA). Ramoplanin tends to stick to nonpolar surfaces such as plastic plates that are usually used for MIC measurements (Scotti et al., 1992, Diagnostic Microbiology and Infectious Dis. 17,: 209-211). Therefore 4% (w/v) of human serum albumin which reflects the serum albumin content of human blood has been added to the media to prevent unspecific binding of ramoplanin. Different molar mixture ratios of the lipoglycodepsipeptide ramoplanin were tested with SBE-β-CD. The Gram-positive strains tested for the cultivation methods were:
Staphylococcus aureus ATCC 29213
Enterococcus faecalis ATCC 29212

The following inocula were incubated:

**Table 3**

| | |
|---|---|
| S. aureus ATCC 29213 | 3.6 * 10⁵ CFU |
| E. faecalis ATCC 29212 | 7.6 * 10⁵ CFU |

| Antibiotic activity (MIC in µg/ml) of ramoplanin as a function of the SBE-β-CD quantity (given is the molar ratio of the quantities) in vitro | | | |
|---|---|---|---|
| Ramoplanin : SBE-β-CD | | | |
| | 1:0 | 1:2 | 1:10 |
| S. aureus ATCC29213 | 0.5 | 0.5 | 0.5 |
| E. faecalis ATCC 29212 | 0.5 | 0.5 | 0.5 |

Surprisingly, the cyclodextrin does not negatively affect in these experiments the antibiotic activity of ramoplanin, although by the molecular interaction of the cyclodextrin with ramoplanin at the same molar ratios the hemolytic property of the lipoglycodepsipeptide can nearly completely be suppressed.

### Example 4: Influence of the acute toxicity of lipoglycodepsipeptides by cyclodextrins in vivo.

The i.v. administration of ramoplanin formulated in 0.9% NaCl showed short term in vivo toxic effects like progressive necrotization at the site of injection and hemolysis as revealed by urine discoloration in animal studies (Parenti et. al. 2000, US patent 6,720,305, Injectable formulations containing ramoplanin, WO00/06119, New Injectable Formulations Containing Ramoplanin).

To assess, whether ramoplanin formulated with cyclodextrin can also reduce toxicity in vivo, the drug was administered intravenously to rats using different formulations (either cyclodextrin or 0.9% NaCl).

Thus, this example shows the effect of β-cyclodextrins on the short term toxicity in rats caused by high concentrations of lipoglycodepsipeptides. Short term toxicity, which is defined as toxic symptoms, e.g. behavioural disorders, within 4 days in living animals and alteration of organs at the fourth day after the first i.v. administration, were investigated. Herein, ramoplanin serves as an example molecule for the antibiotics of the class of the lipoglycodepsipeptides and sulfobutylether-β-cyclodextrin (SBE-β-CD) as examples for modified β-cyclodextrins.

Ramoplanin was dissolved in 0.9 % NaCl solution with and without addition of SBE-β-CD. In the batch with SBE-β-CD, there was a molar ratio (SBE-β-CD:ramoplanin) of 10 : 1. The statements of the ramoplanin concentration (in mg/1) refer to the final concentrations (dose) of ramoplanin in the experiment. The statements of the molar ratio refer to the molecular weight of ramoplanin A2 and the free acid of SBE-β-CD, respectively.

Female Hsd/Cpb:WU Wistar Unilever rats weighing 80 to 100 g were used for this study (Harlan Winkelmann, Germany).

The test compound was administered intravenously for three days once daily by a 1 ml glass syringe at a dosage of 30 mg/kg (3 rats / group). An application volume of 2 ml/kg was administered into the tail vein with a constant speed during a period of 15 seconds. The individual dosages were calculated on basis of the body weight at the time of administration.

At 30 mg/kg ramoplanin formulated with cyclodextrin showed a significantly reduced short term in vivo toxicity effect in comparison to ramoplanin formulated with 0.9% NaCl, e.g. hemolysis was only detected in animals treated with 30 mg/kg ramoplanin formulated in 0.9% NaCl.

In summary this example shows, the formulation of ramoplanin with cyclodextrin reduces significantly short term toxicity effect in vivo, which is in agreement with the results observed in in vitro assays.

## Claims

1. A pharmaceutical composition comprising as an active agent a lipoglycodepsipeptide in a physiologically effective dose and a cyclodextrin or a cyclodextrin derivative.

2. The pharmaceutical composition according to claim 1, wherein the lipoglycodepsipeptide has a structure according to formula I 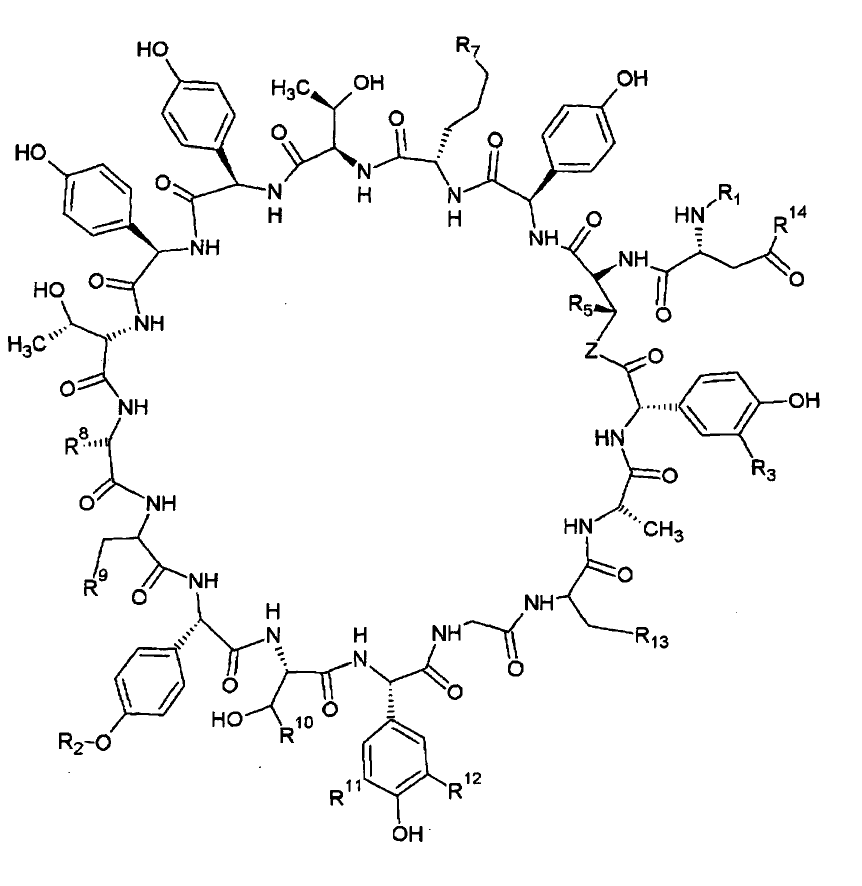 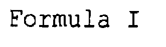
wherein R1 is -H, or a saturated or unsaturated, branched or linear aliphatic, or aromatic, or aliphatic/aromatic acyl residue with 2 to 30 carbon atoms,
wherein R2 is -H or any glycone,
wherein R3 is -H or halogen,
wherein R5 is -H, -CH₃, -COOH, -COO⁻, or -(CO)-NH₂,
wherein R8 is -CH₂-C₆H₅ or -CH₂-CH₂-CH₂-NH-(CO)-NH₂
wherein R9 is -CH₂-CH₂-R6 or 2-iminoimidazolidin-4-yl,
wherein R10 is -H or -CH₃,
wherein R11 and R12 are independently of each other -H or halogen,
wherein R13 is -CH(CH₃)₂ or 2-iminoimidazolidin-4-yl,
wherein R14 is -NH₂ or -OH,
wherein R6 and R7 are independently of each other -NH₃⁺, - NH₂, -NHR4, - -NH₂⁺-R4, wherein R4 is -Ac, -C(NH)NH₃⁺, or saturated or unsaturated, branched or linear C1-C12-alkyl,
and
wherein Z is -O-, -NH-, or -NH-CH₂- (amide bond).

3. The pharmaceutical composition according to claim 1 or 2, wherein the lipoglycodepsipeptide is selected from the group consisting of "ramoplanin A1, ramoplanin A2, ramoplanin A3, ramoplantose, ramoplanin-like derivatives, janiemycin, enduracidins, synthetic amide-derivatives of the aforementioned compounds, and salts of the aforementioned compounds".

4. The pharmaceutical composition according to one of claims 1 to 3, comprising several different lipoglycodepsipeptides in a physiologically effective dose each.

5. The pharmaceutical composition according to one of claims 1 to 4, wherein the lipoglycodepsipeptide is present as an alkali or alkaline earth salt, preferably as a Na or calcium salt, in particular as a di-calcium salt (Ca₂Cl₂ salt), or as an ammonium salt, or wherein the lipoglycodepsipeptide is neutral, or wherein the lipoglycodepsipeptide is present as a cationic part of a salt, wherein in the last alternative as a counter ion preferably an ion from the group comprising "hydrochloride, sulfonate, nitrate, phosphate, succinate, malate, maleate, citrate, tartrate, lactate, gluconate and sulfonate" can be employed.

6. The pharmaceutical composition according to one of claims 1 to 5, comprising the lipoglycodepsipeptide in a total quantity from 0.001 to 20 wt.%, in particular from 0.05 to 20 wt.%, preferably from 0.1 to 5 wt.%.

7. The pharmaceutical composition according to one of claims 1 to 6, wherein the cyclodextrin or cyclodextrin derivative is an α or β-cyclodextrin and preferably has the general formula II 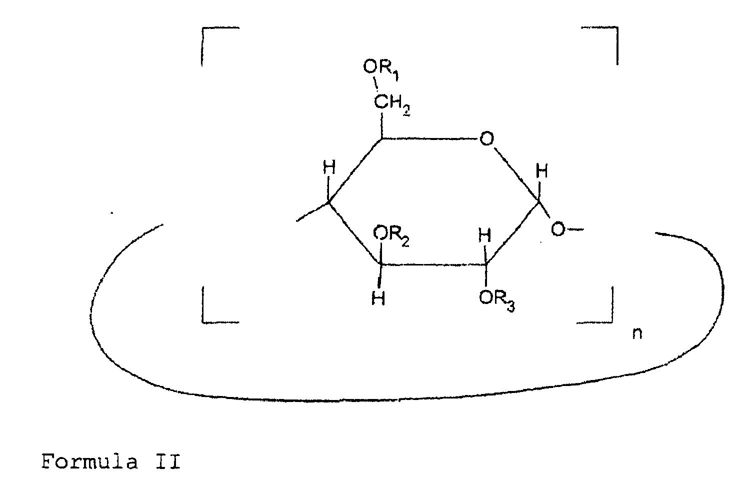
wherein R1, R2, and R3 may be identical or different and an arbitrary physiologically tolerated residue, preferably -H, C1-C8 alkyl, -SO₂OH, -PO(OH)₂, or -CO-R4 with R4 = Cl-C8 alkyl, wherein the C1-C8 alkyl may be single or multiple at identical or at different C atoms with -OH, - COOH, -CONHR5, -NHCOR6, -SO₂OH, -PO(OH)₂, or tetrazol-5-yl with R5 = -H or C1-C4 alkyl and R6 = carboxylphenyl,
wherein n = 6 or 7
wherein R1, R2 and R3 may be randomized in different glucopyranose units,
wherein an oxygen atom or several oxygen atoms of the glucopyranose units, in particular the oxygen atom at C6, may be substituted by sulfur atoms,
including physiologically tolerated salts of such cyclodextrins.

8. The pharmaceutical composition according to one of claims 1 to 7, wherein the cyclodextrin or cyclodextrin derivative is selected from the group comprising "α-cyclodextrin, β-cyclodextrin, hydroxy-(C1-C8 alkyl)-α-cyclodextrin, hydroxy-(C1-C8 alkyl)-β-cyclodextrin, (2-hydroxypropyl)-β-cyclodextrin, (2-hydroxypropyl)-α-cyclodextrin, sulfo-(C1-C8 alkyl)-ether-α-cyclodextrin, sulfo-(C1-C8 alkyl)-ether-β-cyclodextrin, sulfobutylether-α-cyclodextrin, sulfobutylether-β-cyclodextrin".

9. The pharmaceutical composition according to one of claims 1 to 8, comprising the cyclodextrin or cyclodextrin derivative in a quantity from 0.01 to 99 wt.%, in particular from 0.1 to 95 wt.%, preferably 5 to 95 wt.%.

10. The pharmaceutical composition according to one of claims 1 to 9, comprising further additional and/or auxiliary substances, in particular galenic auxiliary substances.

11. The pharmaceutical composition according to claim 10, comprising
A) 0.001 to 20 wt.%, in particular 0.05 to 20 wt.%, preferably 0.1 to 20 wt.% lipoglycodepsipeptide,
B) 0.001 to 99.899 wt.%, in particular 0.01 to 95 wt.%, preferably 0.1 to 20 wt.% cyclodextrin or cyclodextrin derivative,
C) 0.1 to 99.998 wt.%, in particular 50 to 99.94 wt.%, preferably 90 to 99.8 wt.% additional and/or auxiliary substances and optionally dilution agents,
wherein the components A) to C) always add up to 100 %.

12. The use of a pharmaceutical composition according to one of claims 1 to 11 for the production of a drug for the treatment and/or prophylaxis of viral and/or bacterial and/or parasitary infectious diseases and/or of fungal diseases.

13. The use according to claim 12, wherein the drug is galenically prepared for the oral administration or for the injection by mixture with galenic auxiliary and carrier substances.

14. A method for the treatment of a bacterial, viral or parasitary infectious disease and/or a fungal disease, wherein a person, which has fallen ill with the disease or is in danger of falling ill therewith, is administered a physiologically effective dose of a pharmaceutical according to one of claims 1 to 11.

15. The method according to claim 14, wherein the person is administered a daily dose from 1 to 50,000 mg, preferably 50 to 30,000 mg, most preferably from 100 to 20,000 mg lipoglycodepsipeptide over a period from 1 to 60 days, preferably 1 to 30 days.

16. A packing unit with a multitude of administration units, wherein each administration unit is prepared for the administration within a treatment plan according to one of claims 14 or 15.

17. The method for the production of a pharmaceutical composition according to one of claims 1 to 11, wherein the lipoglycodepsipeptide in a physiologically effective dose is mixed with the cyclodextrin or cyclodextrin derivative in a molar ratio lipoglycodepsipeptide/cyclodextrin from 10:1 to 1:500, preferably 5:1 to 1:50, most preferably 1:1 to 1:30, optionally under addition of additional and/or auxiliary substances in galenically usual additions.
